## Europäisches Patentamt

(19) **European Patent Office**

Office européen des brevets

(11) Publication number: **0 110 574**
**B1**

## EUROPEAN PATENT SPECIFICATION

(12)

(45) Date of publication of patent specification: **13.07.88**

(21) Application number: **83306601.2**

(22) Date of filing: **28.10.83**

(51) Int. Cl.⁴: **C 12 P 19/20,** C 12 N 11/08,
C 13 K 1/06

(54) Process for preparing high-dextrose starch hydrolysates with immobilized glucoamylase.

(30) Priority: **29.10.82 GB 8231040**

(43) Date of publication of application:
**13.06.84 Bulletin 84/24**

(45) Publication of the grant of the patent:
**13.07.88 Bulletin 88/28**

(84) Designated Contracting States:
**AT BE DE FR IT NL SE**

(56) References cited:
**GB-A-1 538 823**
**GB-A-2 049 698**
**US-A-4 226 937**

**CHEMICAL ABSTRACTS, vol. 95, no. 12,**
**December 1981, page 523, no. 202090b,**
**Columbus, Ohio, USA**

**CHEMICAL ABSTRACTS, vol. 91, no. 11, 10th**
**September 1979, page 606, no. 89507v,**
**Columbus, Ohio, USA V. KRASNOBAJEW:**
**"PAG-B-immobilized glucoamylase: glucose**
**production from soluble starch"**

(73) Proprietor: **CPC INTERNATIONAL INC.**
**International Plaza P.O. Box 8000**
**Englewood Cliffs New Jersey 07632 (US)**

(72) Inventor: **Stouffs, Robert H.M.**
**336 Boulevard du Souverain**
**B-1160 Brussels (BE)**
Inventor: **Venker, Henry Paul**
**Deken Dewinterstraat 32**
**B-2600 Berchem Antwerp (BE)**

(74) Representative: **Pennant, Pyers et al**
**Stevens, Hewlett & Perkins 5 Quality Court**
**Chancery Lane**
**London, WC2A 1HZ (GB)**

Courier Press, Leamington Spa, England.

**Description**

This invention relates to a process for obtaining starch hydrolysates having high (at least 93% and normally 95% or more) dextrose contents by means of immobilized glucoamylase. Throughout the specification and claims all percentages are by weight, dry basis, unless stated otherwise.

Dextrose is conventionally obtained from starch by saccharifying, in batch, a thinned or partially converted starch with soluble glucoamylase; the conventional processes normally produce final hydrolysates containing 95%, or slightly more, dextrose.

Within recent years many enzymatic processes have been greatly improved by using the enzyme in immobilized, rather than soluble, form. Immobilized enzyme techniques permit reactions to be conducted on a continuous basis (by passing continuously the substrate through a bed or column of the immobilized enzyme), they make more efficient use of the enzymes, they permit more precise control of the processes and the end products, they generally require less refining and are frequently lower in energy requirements than processes employing soluble enzymes.

Naturally, a great deal of work has been done in the area of immobilizing glucoamylase for the production of dextrose from starch, but most reported processes give lower dextrose levels than can be obtained by convention methods, i.e. 93% dextrose or less as opposed to the 95% or more obtainable with the batch processes using soluble glucoamylase.

Three recently issued patents on this subject (which discloses a large number of other patent and journal articles in the field) are U.S. Patent 4011137 to Thompson et al., U.S. Patent 4132595 to Hebeda et al. and U.S. Patent 4202939 to Mueller et al. U.S. 4011137 discloses converting starch to dextrose with an enzyme system comprising immobilized glucoamylase and immobilized alpha-amylase plus, optionally, pullulanase. The examples, all of which exemplify batch rather than continuous processes, show final dextrose levels of from about 92% to as high as 96.7%, depending upon the type of alpha-amylase used, the ratio of alpha-amylase to glucoamylase, and the presence or absence of pullulanase.

U.S. Patent 4132595 teaches a process whereby a thinned or partially converted starch (DE 2—20) is saccharified first with soluble glucoamylase to a DE of 30 to 85 and the resulting hydrolysate is then passed through a mass of immobilized glucoamylase for the final saccharification. Dextrose levels of at least 93% and as high as about 97% are obtainable by this technique but the process cannot, of course, be adapted to a completely continuous operation and the highest dextrose levels are achieved only with relatively dilute substrates, i.e. having solids levels of 5% to 10%.

In accordance with U.S. Patent 4202939 glucoamylase is immobilized by complexing it with colloidal silica in a specific manner and the resulting enzyme composite is used to hydrolyse thinned or partially hydrolysed starch to dextrose. The highest dextrose level reported in the patent is 93.1±0.4% using a 71 DE starch hydrolysate as the substrate.

Published British Application GB 2049698 A to CPC International Inc. also relates to immobilized glucoamylase but the process described therein produces non-crystallizing syrups containing both maltose (at least 20%) and dextrose (not more than 44 to 48%), any balance being higher saccharides (having degrees of polymerisation of 3 and greater), by continuously passing a maltose-containing starch hydrolysate through a mass of immobilized glucoamylase. There is no suggestion in the British Patent Application that such a technique could be used to produce hydrolysates containing very high levels of dextrose.

The present invention comprises a process for producing dextrose from starch by contacting a starch hydrolysate with immobilized glucoamylase under saccharifying conditions, characterized in that the starch hydrolysate has a DE of at least 40 and a maltose content of at least 45% and the time of contact is sufficient to produce a product containing at least 93% dextrose.

The critical aspect of the invention is the use of a maltose-containing hydrolysate is defined above as the substrate, and the process is not dependent upon any specific method of immobilizing the glucoamylase or upon the carrier employed, although naturally one should use an immobilized enzyme system wherein the glucoamylase is, and remains, both active and insoluble under the process conditions for a reasonable period of time.

For reasons of economy and simplicity it is preferred to immobilize the glucoamylase by absorption onto a suitable porous carrier, and there are many such materials on the market. Porous phenolic resins having anion-exchange capacity such as Duolite ES 568 and Duolite ES 562, both manufactured by Diamond Shamrock, have been found to be very suitable.

The saccharification can be conducted batch wise by simply adding the immobilized glucoamylase to a tank containing the maltose-containing substrate but the greatly preferred method is to operate the process continuously by loading the immobilized enzyme into a receptacle, preferably a column, and passing the substrate therethrough. As will be noted from the examples, such a continuous process can be conducted over very long time periods, and substrates containing at least 20%, and up to 30% solids can be used, thereby rendering the process extremely practical for industrial application.

Although any hydrolysate having a DE of at least 40 and a maltose content of at least 45% is suitable in the practice of the invention, the preferred substrate is a starch hydrolysate prepared by hydrolysing a thinned or partially converted starch having a DE of 20 or lower (and most preferably having been converted to a DE of between 12 and 20 by an anzyme such as alpha-amylase) with beta-amylase. Other,

2

but less preferred methods of preparing the substrate include hydrolysing an acid-thinned starch with beta-amylase or hydrolysing an enzyme-thinned starch with beta-amylase or another maltogenic enzyme plus pullulanase (thereby producing a substrate having a very high maltose content) or with a fungal amylase. Operable substrates can be purchased on the open market or can be easily prepared on the premises. The fact that the most preferred substrate, i.e. the one which will yield the highest dextrose levels (as illustrated in Example 1 to follow), is extremely simple and inexpensive to prepared renders the process of the operation exceptionally suitable for an industrial operation.

The glucoamylase preparation used in our examples was a commercially available product derived from *Aspergillus niger* (the most common source of this enzyme), but any glucoamylase can be employed. Of course, optimum saccharifying conditions (i.e. temperature and pH) as well as immobilization techniques providing the maximum binding and retention of the enzyme may vary depending upon the specific glucoamylase employed. Selection of the best conditions for any particular enzyme preparation is well within the competence of the skilled operator. The preparation should be low in transglucosidase activity, and most commercially available glucoamylase preparations meet this criterion. When using such a preparation no special pre-treatment to remove other enzymes prior to immobilization is necessary.

The pH during the process should normally between 3.0 and 7.0, the operable range for most glucoamylase preparations. In conventional processes using soluble glucoamylase the optimum pH is known to be about 4.2 or within the range of 4.2 to 4.5, and most reported processes using immobilized glucoamylase also recommend this pH range. Surprisingly, in the process of the present invention the highest dextrose levels are obtained with a pH in the range of 3 to 4, preferably about 3.5.

The time of contact of the substrate with the immobilized glucoamylase is, of course, important for the production of high dextrose levels, which contact time is regulated in a column or other continuous operation by the flow rate of the substrate. As is shown in the examples, dextrose levels of 95% and higher can be achieved, and maintaind, by appropriate regulation of the flow rate.

The temperature during the process should normally be within the range of about 40°C and about 70°C, preferably between 45°C and 55°C. During a lengthy continuous operation one can, of course, obtain constant product quality by increasing the temperature instead of, or in addition to, reducing the flow rate.

As mentioned previously, substrates having solids concentrations of 20% to 30% (which are similar to those currently used in batch processes) can be used; as would be expected, and as is the case in batch processing, higher dextrose levels are achieved with lower concentrations, all other conditions being equal.

The following examples will illustrate the practice of the invention. They are presented for illustrative purposes only and should not be construed as limiting the claims.

## Example 1
### Comparison of Various Substrates, Both Within and Outside of the Scope of the Invention

A double-jacket glass column having dimensions of 4.5 × 40 cm was packed with 600 ml of Duolite ES—568 resin, a porous weak anion-exchange resin of the phenol-formaldehyde type, produced by Diamond Shamrock Co. The resin was washed upflow with demineralised water at 70°C for half an hour to remove any entrapped air bubbles. Then, the packed resin was washed downflow at room temperature with 0.1 M acetate buffer pH 4.2 for 1 hour at the rate of 4 BVH (bed volumes per hour). Next, a glucoamylase solution was offered at an enzyme load of 8 units per ml. carrier and recirculated for 24 hours at a flowrate of 4 BVH in order to immobilize the enzyme on the resin. After that, the resin was washed with demineralised water at a rate of 4 BVH for one hour and the effluent collected for bound enzyme determination. Assuming one unit of glucoamylase to be the amount of enzyme activity producing from a 4% d.s. maltodextrin of 20 D.E. one gram per hour of reducing sugars at pH 4.3 and 60°C, the amount of bound enzyme was then calculated using the equation:

$$\text{Bound enzyme, \%} = 100 - \frac{(\text{U/ml measured in effluent} \times 2400 \times 100)}{\text{enzyme offered, U}}$$

A binding of 98.6% was determined for the column.

Next, the loaded resin was quantitatively transferred to a 1 liter beaker where it was stirred to ensure homogeneity. After that, the carrier-enzyme was divided into 60 ml portions to pack 10 individual columns of dimensions 2.5 × 30 cm. Each of these columnns was then fed with a specific starch hydrolysate at 20% dry substance. The starch hydrolysates investigated as feed were:

a) 12 DE enzyme thinned corn starch.

b) 20 DE enzyme thinned corn starch.

c) 30 DE maltose syrup obtained by conversion with beta-amylase of a 2 DE enzyme thinned corn starch.

d) 30 DE maltose syrup obtained by conversion with beta-amylase of a 5 DE enzyme thinned corn starch.

e) 42 DE high maltose syrup obtained by conversion with beta-amylase of a 12—20 DE enzyme thinned corn starch.

## 0 110 574

f) 42 DE maltose-containing syrup obtained by conversion with beta-amylase of an acid thinned corn starch.

g) 42 DE syrup obtained by alpha-amylase conversion of a 20 DE enzyme thinned corn starch.

h) 50 DE syrup obtained by fungal alpha-amylase conversion of a 20 DE enzyme thinned corn starch.

i) 50 DE high maltose syrup obtained by beta-amylase/pullulanase conversion of an enzyme thinned corn starch.

j) 82 DE high dextrose syrup obtained by preliminary conversion with free glucoamylase of a 20 DE maltodextrin (in accordance with U.S. Patent 4132595).

These starch hydrolysates were clarified but not demineralised. Some 150 ppm $SO_2$ were added to the feed and pH was adjusted to 4.2 with hydrochloric acid. The various columns were sweetened on with their respective feeds for one hour at room temperature and the column temperatures were then raised to 50°C and the flowrates adjusted to 3 BVH. After at least 10 bed volumes were passed on the columns, a sample was taken and the sugar breakdown was analysed. Next, the flowrates were adjusted to 2 BVH. Again, at least 10 bed volumes were passed on the columns before taking a sample. The same procedure was repeated for 1 and 0.5 BVH.

Table No. 1 shows the results obtained with the various feeds. As can be seen from the Table, feeds e, h and i, which illustrate the practice of the invention, all produced products containing at least 93% dextrose when the process was operated at 0.5 BVH, which dextrose levels were higher than those achieved using any of the other feeds with the exception of j, which illustrates the process of U.S. Patent 4132595. It will also be noted, of course, that feed e, prepared by hydrolysing a 12 to 20 DE enzyme thinned corn starch with beta-amylase, gave the best results. One can only speculate as to why feed e produces so much more dextrose than, for example, feeds c and d which contain almost the same amount of maltose, and feed f which contains approximately the same amount of saccharides having degrees of polymerisation of 4 and greater plus a relatively high amount of maltose, i.e. 41.5%.

One can also only speculate why feeds h and i, having quite different saccharide breakdowns, produce about the same amount of dextrose, and also about thee same amount as feed j which has an entirely different saccharide breakdown. Whatever mechanism or mechanisms are responsible, the data clearly show the production of high dextrose yields using substrates having DE's of at least 40 and maltose contents of at least 45%.

TABLE 1

| | FEED | | | | DEXTROSE CONTENT IN PRODUCT | | | |
|---|---|---|---|---|---|---|---|---|
| | DP1 | DP2 | DP3 | ≥DP4 | AT 3 BVH | AT 2 BVH | AT 1 BVH | AT 0.5 BVH |
| a) 12 DE E | — | — | — | — | 80.9 | 84.1 | 88.7 | 91.5 |
| b) 20 DE E | 2.4 | 6.5 | 7.2 | 83.9 | 84.5 | 86.6 | 89.8 | 91.5 |
| c) 30 DE E/E (from 2 DE) | 0.4 | 48.9 | 4.9 | 45.8 | 79.2 | 82.6 | 86.1 | 89 |
| d) 30 DE E/E (from 5 DE) | 0.5 | 47.6 | 7.1 | 44.8 | 82.9 | 84.7 | 89.4 | 90.3 |
| e) 42 DE E/E HM | 1.5 | 51.0 | 18.5 | 29.0 | 83.8 | 90.2 | 93.5 | 94.5 |
| f) 42 DE A/E | 7.6 | 41.5 | 17.7 | 27.4 | 80.9 | 88.9 | 90.5 | 89.6 |
| g) 42 DE E/E alpha-amylase | 11.0 | 24.0 | 20.5 | 44.5 | 80.3 | 89.4 | 91.3 | 92.2 |
| h) 50 DE E/E fungal alpha | 8.5 | 58.5 | 15.1 | 17.9 | 74.0 | 90.7 | 92.9 | 93.2 |
| i) 54 DE E/E HM | 2.0 | 79.0 | 13.0 | 6.0 | 88.5 | 91.0 | 92.8 | 93.0 |
| j) 82 DE E/E | 77.1 | 5.9 | 0.2 | 16.8 | 90.0 | 90.9 | 92.2 | 93.5 |

DP = Degree of polymerisation

### Example 2

To demonstrate that the process of the invention is improved by using lower pH in continuous processing than currently practiced in batch operation, two 200 ml columns were run in parallel. One was operated at pH 4.2, which is the preferred pH in batch processing, and the other one at pH 3.5. The general loading procedure of Example 1 was followed, and the substrate used was hydrolysate e, of that example.

Table No. 2 shows the results obtained. As can be seen, less isomaltose is formed at pH 3.5. This decreased reversion tends to increase the dextrose level in the final product. Also, it demonstrates that during an extensive period of time, a dextrose level can be obtained which is higher than 95%, and by appropriate regulation of column flowrate, higher than 96%.

### Example 3

To demonstrate that the process of this invention can be used at various dry substances, parallel runs were made with substrate e, at 20% and 30% d.s., in 200 ml columns. The general loading procedure of Example 1 was followed, with the exception that feed pH was regulated at 3.5.

The results are set forth in Table No. 3. These results demonstrate that a dextrose content of at least 95% can also be obtained at 30% d.s., even after the long operation time of about 3400 hours. Careful adjustment of flowrates would maintain the dextrose level constant at 95+% dextrose throughout column life. The data show that the maximum dextrose level obtained at 30% d.s. would be slightly less than the maximum dextrose level obtained at 20% d.s. under identical operating conditions. This influence of d.s. on maximum dextrose level is also experienced in batch processing.

TABLE 2

| OPERATION TIME (hours) | pH 3.5 | | | pH 4.2 | | |
|---|---|---|---|---|---|---|
| | BVH | DEXTROSE % | ISOMALTOSE % | BVH | DEXTROSE % | ISOMALTOSE % |
| 18 | 0.88 | 95.1 | 0.6 | 0.95 | 94.9 (a) | 0.9 |
| 192 | 0.79 | 96.3 | 0.5 | 0.87 | 95.2 (a) | 0.5 |
| 336 | 0.83 | 96.5 | 0.7 | 0.87 | 95.9 (a) | 0.8 |
| 456 | 0.89 | 96.2 | 0.5 | 0.80 | 96.0 (a) | 0.9 |
| 648 | 0.90 | 95.5 | 0.3 | 0.71 | 95.6 (a) | 0.8 |
| 840 | 0.66 | 96.3 | 0.9 | 0.60 | 95.9 (a) | 1.2 |
| 1008 | 0.59 | 96.3 | 1.0 | 0.49 | 95.8 (a) | 1.6 |
| 1200 | 0.61 | 96.4 | 0.4 | 0.49 | 96.1 (a) | 1.0 |

(a) The listed values are somewhat higher than the value obtained under identical conditions in Example 1. This was due to more adequate regulation of flowrates versus enzyme load and column life time.

TABLE 3

| OPERATION TIME, hours | 20% d.s. | | | 30% d.s. | | |
|---|---|---|---|---|---|---|
| | BVH | DEXTROSE % | ISOMALTOSE % | BVH | DEXTROSE % | ISOMALTOSE % |
| 192 | 1.03 | 96.1 | 0.3 | 0.71 | 94.9 | 0.7 |
| 360 | 0.90 | 96.1 | 0.6 | 0.62 | 95.1 | 0.8 |
| 672 | 0.75 | 97.2 | 0.5 | 0.49 | 96.0 | 1.3 |
| 936 | 0.68 | 96.0 | 0.7 | 0.49 | 96.0 | 1.3 |
| 1200 | 0.60 | 95.5 | 0.8 | 0.52 | 94.1 | 1.0 |
| 1680 | 0.43 | 96.0 | 1.3 | 0.54 | 94.1 | 0.9 |
| 1872 | 0.36 | 96.3 | 0.7 | 0.38 | 94.5 | 0.9 |
| 2328 | 0.36 | 96.0 | 1.2 | 0.37 | 95.2 | 1.4 |
| 3144 | 0.43 | 95.8 | 0.3 | 0.27 | 95.8 | 1.0 |
| 3384 | 0.19 | 96.1 | 0.8 | 0.19 | 95.0 | 1.0 |

Example 4

To ascertain the influence of the amount of offered enzyme and to demonstrate that the productivity can be increased by using higher amounts of offered enzyme, a column was loaded in accordance with the procedure described in Example 1, but 20 A.U. glucoamylase were offered to the carrier, and the column was operated at pH 3.5, with a feed (hydrolysate e) at 20% d.s. The results are shown in Table 4.

TABLE 4

| Operating Time (Hours) | BVH | Dextrose | Isomaltose |
|---|---|---|---|
| 264 | 1.15 | 95.8 | 2.0 |
| 600 | 1.18 | 96.4 | 1.1 |
| 936 | 1.26 | 96.1 | 1.1 |
| 1656 | 1.24 | 95.8 | 0.6 |
| 2184 | 0.83 | 96.1 | 0.7 |
| 2544 | 0.70 | 96 | 0.8 |
| 2976 | 0.40 | 96.9 | 1.3 |
| 3672 | 0.24 | 95.9 | 1.5 |
| 4152 | 0.24 | 96.3 | 1.4 |
| 4320 | 0.23 | 95.9 | 0.7 |

**Claims**

1. Process for producing dextrose from starch by contacting a starch hydrolysate with immobilized glucoamylase under saccharifying conditions, characterized in that the starch hydrolysate has a DE of at least 40 and a maltose content of at least 45% and the time of contact is sufficient to produce a product containing at least 93% dextrose.

7

2. Process of Claim 1 characterized in that the starch hydrolysate has been prepared by hydrolyzing a partially converted starch with beta-amylase.

3. Process of Claims 1 or 2 characterized in that the starch hydrolysate has been prepared by partially converting starch with alpha-amylase to a DE of not greater than 20 and then hydrolysing with beta-amylase.

4. Process of any of the preceding claims characterized in that the hydrolysate is contacted with the immobilized glycoamylase by passing the hydrolysate through a column containing the immobilized glucoamylase.

5. Process of any of the preceding claims characterized in that the starch hydrolysate is contacted with the immobilized glucoamylase at a pH of between 3 and 7.

6. Process of any of the preceding claims characterized in that the starch hydrolysate is contacted with the immobilized glucoamylase at a pH of between 3 and 4.

7. Process of any of the preceding claims characterized in that the dry substance concentration of the hydrolysate is at least 20%.

8. Process of any of the preceding claims characterized in that the glucoamylase has been immobilized by absorption onto a porous carrier.

9. Process of any of the preceding claims characterized in that the glucoamylase has been immobilized by absorption onto a porous phenolic resin.

## Patentansprüche

1. Verfahren zur Herstellung von Dextrose aus Stärke durch Kontaktieren eines Stärkehydrolysats mit immobilisierter Glucoamylase unter verzuckernden Bedingungen, dadurch gekennzeichnet, daß das Stärkehydrolysat ein DE von zumindest 40 und einen Maltosegehalt von zumindest 45% hat und die Kontaktzeit ausreichend ist, damit ein Produkt gebildet wird, das zumindest 93% Dextrose enthält.

2. Verfahren nach Anspruch 1, dadurch gekennzeichnet, daß das Stärkehydrolysat durch Hydrolse einer teilweise umgewandelten Stärke mit beta-Amylase hergestellt wird.

3. Verfahren nach Anspruch 1 oder 2, dadurch gekennzeichnet, daß das Stärkehydrolysat durch teilweise Umwandlung der Stärke mit alpha-Amylase zu einem DE von nicht mehr als 20 und anschließendes Hydrolysieren mit beta-Amylase hergestellt wird.

4. Verfahren nach einem der vorgenannten Ansprüche, dadurch gekennzeichnet, daß das Hydrolysat mit der immobilisierten Glucoamylase kontaktiert wird, indem das Hydrolysat durch eine Säule läuft, die die immobilisierte Glucoamylase enthält.

5. Verfahren nach einem der vorgenannten Ansprüche, dadurch gekennzeichnet, daß das Stärkehydrolysat mit der immobilisierten Glucoamylase bei einem pH-Wert zwischen 3 und 7 kontaktiert wird.

6. Verfahren nach einem der vorgenannten Ansprüche, dadurch gekennzeichnet, daß das Stärkehydrolysat mit der immobilisierten Glucoamylase bei einem pH-Wert zwischen 3 und 4 kontaktiert wird.

7. Verfahren nach einem der vorgenannten Ansprüche, dadurch gekennzeichnet, daß die Trockensubstanz-Konzentration des Hydrolysats zumindest 20% beträgt.

8. Verfahren nach einem der vorgenannten Ansprüche, dadurch gekennzeichnet, daß die Glucoamylase durch Absorption auf einen porösen Träger immobilisiert wird.

9. Verfahren nach einem der vorgenannten Ansprüche, dadurch gekennzeichnet, daß die Glucoamylase durch Absorption auf ein poröses Phenolharz immobilisiert wird.

## Revendications

1. Procédé de production de dextrose à partir d'amidon par mise en contact d'un hydrolysat a'amidon avec de la gluco-amylase immobilisée, dans des conditions de saccharification, caractérisé en ce que l'hydrolysat d'amidon a un équivalent de dextrose d'au moins 40 et une teneur en maltose d'au moins 45%, et la durée de contact est suffisante pour former un produit contenant au moins 93% de dextrose.

2. Procédé suivant la revendication 1, caractérisé en ce que l'hydrolysat d'amidon a été préparé par hydrolyse par la bêta-amylase d'un amidon partiellement saccharifié.

3. Procédé suivant la revendication 1 ou 2, caractérisé en ce que l'hydrolysat d'amidon a été préparé par saccharification partielle d'amidon avec de l'alpha-amylase jusqu'à un équivalent de dextrose ne dépassant pas 20, puis hydrolyse à la bêta-amylase.

4. Procédé suivant l'une quelconque des revendications précédentes, caractérisé en ce que l'hydrolysat est mis en contact avec la gluco-amylase immobilisée par passage de l'hydrolysat sur une colonne contenant la gluco-amylase immobilisée.

5. Procédé suivant l'une quelconque des revendications précédentes, caractérisé en ce que l'hydrolysat d'amidon est mis en contact avec la gluco-amylase immobilisée à un pH comprise entre 3 et 7.

6. Procédé suivant l'une quelconque des revendications précédentes, caractérisé en ce que l'hydrolysat d'amidon est mis en contact avec la gluco-amylase immobilisée à un pH compris entre 3 et 4.

7. Procédé suivant l'une quelconque des revendications précédentes, caractérisé en ce que la concentration en substance sèche de l'hydrolysat est au moins égale à 20%.

8. Procédé suivant l'une quelconque des revendications précédentes, caractérisé en ce que la gluco-amylase a été immobilisée par absorption sur un support poreux.

9. Procédé suivant l'une quelconque des revendications précédentes, caractérisé en ce que la gluco-amylase a été immobilisée par absorption sur une résine phénolique poreuse.